# EUROPÄISCHE PATENTANMELDUNG

(11) **EP 2 801 812 A1**
(43) Veröffentlichungstag der Anmeldung: **12.11.2014**
(21) Anmeldenummer: 13168370.8
(22) Anmeldetag: 17.05.2013
(51) Int. Cl.: G01N 15/14, G01N 15/12

(54) **Düse und Verfahren für die Durchflußzytometrie**

(30) Priorität: 08.05.2013 DE 102013208584
(71) Anmelder: Masterrind GmbH, 27283 Verden (DE); Rath, Detlef, 31535 Neustadt (DE)
(72) Erfinder: Rath, Detlef, 31535 Neustadt (DE)
(74) Vertreter: Taruttis, Stefan Georg

(57) **Zusammenfassung**

Die Erfindung betrifft eine Düse für die Durchflusszytometrie, deren Gehäuse sich zu einem Auslass verjüngt und in dem ein Zuführungsrohr für eine Kemstromflüssigkeit angeordnet ist, dessen Austrittsöffnung in einem Abstand vor dem Auslass des Gehäuses angeordnet ist. Der Auslass des Gehäuses bildet den Auslass der Düse. Das Gehäuse der Düse erstreckt sich von seinem Auslass, der an dessen ersten Ende angeordnet ist, zu seinem gegenüberliegenden zweiten Ende und weist einen mit dem Innenvolumen verbundenen Einlass für eine Hüllstromflüssigkeit auf. Die Düse zeichnet sich dadurch aus, dass sich in dem Gehäuse beidseitig des Zuführungsrohrs ein Leitelement erstreckt, das die Ausrichtung von Partikeln begünstigt.

## Beschreibung

Die vorliegende Erfindung betrifft eine Düse für ein Durchflußzytometer, ein Durchflußzytometer mit der Düse und ein Verfahren, das mit der Düse bzw. mit einem die Düse aufweisenden Durchflußzytometer durchführbar ist, zur Ausrichtung von Partikeln in einem Flüssigkeitsstrom, der einen partikelhaltigen Kernstrom innerhalb eines Hüllstroms aufweist oder daraus besteht. Insbesondere betrifft die Erfindung die Verwendung der Düse in einem Verfahren zur Ausrichtung von Partikeln und Sortierung der Partikel in Abhängigkeit von einer Eigenschaft, die im Anschluss an den Durchtritt der Partikel durch die Düse detektiert wird. Dabei ist die Sortierung vorzugsweise das Ablenken von Abschnitten des Flüssigkeitsstroms, insbesondere von Tropfen, die aus dem Flüssigkeitsstrom gebildet sind, in zumindest zwei Fraktionen. In dieser Ausführungsform betrifft die Erfindung die Herstellung von einer Fraktion von Partikeln durch Ausrichtung der Partikel in einem Flüssigkeitsstrom mit der erfindungsgemäßen Düse.

Bevorzugt sind die Partikel, die mit dem Verfahren ausgerichtet und optional in Fraktionen sortiert werden, von insgesamt flacher Gestalt, und sind beispielsweise zu einer Schnittebene symmetrisch, sodass die Partikel senkrecht zu ihrer Längsachse einen Querschnitt mit einer ersten und einer zweiten Dimension aufweisen, wobei der Querschnitt in der ersten Dimension kleiner ist als in der zweiten Dimension. Bevorzugte Partikel sind biologische Zellen, beispielsweise Blutzellen, insbesondere plättchenförmige Zellen und nicht-menschliche Säugetierspermien, insbesondere von einem männlichen Tier gewonnene Spermien, das insbesondere ein Rind, Schwein, Schaf, Elefant, Kamel, Pferd oder ein Ziegenbock ist.

### Stand der Technik

Die US 6,149,867 beschreibt eine gattungsgemäße Vorrichtung und ein Verfahren mit einer Düse zur Sortierung von Säugerspermien in geschlechtschromosomenspezifische Spermienfraktionen. Das Verfahren verwendet ein Durchflusszytometer mit einer Düse, deren Gehäuse konisch zuläuft und in dem eine Zuleitung für die Spermien enthaltende Kernflüssigkeit enthalten ist, sodass am Düsenauslass ein Spermien enthaltender Kernstrom von einem Hüllstrom umgeben austritt.

Die WO 99/05504 beschreibt eine Düse zur Verwendung in einem Durchflusszytometer zur Sortierung von Spermien, die sich dadurch auszeichnet, dass das Gehäuse der Düse in dem Abschnitt zwischen der Auslassöffnung eines Zuführröhrchens für Spermien enthaltende Kernstromflüssigkeit eine im Wesentlichen trichterförmig zulaufende Innenoberfläche hat, die einen ersten ellipsenförmigen Querschnitt hat, und einen daran angrenzenden axialen Abschnitt mit ellipsenförmigem Querschnitt, der um 90° zu dem ellipsenförmigen Querschnitt des stromaufwärts gelegenen Abschnitts gedreht ist.

Die EP 1238261 B1 beschreibt eine Düse für ein Durchflusszytometer zur Sortierung von Spermien, bei dem ein in einer Düse koaxial angeordnetes Zuführröhrchen für Spermien enthaltende Kernstromflüssigkeit von einem Abschnitt mit zylindrischem Außendurchmesser zu einem im Wesentlichen rechteckigen Querschnitt innerhalb eines kegelstumpfförmigen Abschnitts der Düse zuläuft, wobei sich stromabwärts dieses Zulaufröhrchens ein sich verjüngender Abschnitt der Düse mit elliptischem Querschnitt anschließt.

### Aufgabe der Erfindung

Gegenüber dem Stand der Technik liegt die Aufgabe der Erfindung darin, eine alternative Düse zur Ausrichtung von Partikeln in einem Flüssigkeitsstrom und ein Verfahren zur Ausrichtung nicht rotationssymmetrischer Partikel in einem Flüssigkeitsstrom bereitzustellen.

### Allgemeine Beschreibung der Erfindung

Die Erfindung löst die Aufgabe mit den Merkmalen der Ansprüche, und insbesondere mit einer Düse, deren Gehäuse sich zu einem Auslass verjüngt und in dem ein Zuführungsrohr für eine Kernstromflüssigkeit angeordnet ist, dessen Austrittsöffnung in einem Abstand vor dem Auslass des Gehäuses angeordnet ist. Der Auslass des Gehäuses bildet den Auslass der Düse. Das Gehäuse der Düse erstreckt sich von seinem Auslass, der an dessen ersten Ende angeordnet ist, zu seinem gegenüberliegenden zweiten Ende und weist einen mit dem Innenvolumen verbundenen Einlass für eine Hüllstromflüssigkeit auf. Das zweite Ende des Gehäuses kann mit einem Deckel überdeckt sein, in dem optional der Einlass für Hüllstromflüssigkeit angeordnet ist. Bevorzugt ist der Innenquerschnitt des Gehäuses rotationssymmetrisch bzw. kreisförmig und wird entsprechend durch eine rotationssymmetrische Innenfläche gebildet, die zumindest abschnittsweise kegelförmig zum Auslass zuläuft. Das innerhalb des Gehäuses angeordnete Zuführungsrohr ist bevorzugt koaxial zur Längsachse des Gehäuses angeordnet. Die Eintrittsöffnung des Zuführungsrohrs ist mit einer Zuleitung für eine partikelhaltige Kernstromflüssigkeit verbunden.

Die erfindungsgemäße Düse zeichnet sich dadurch aus, dass sich in dem Gehäuse beidseitig des Zuführungsrohrs ein Leitelement mit einem Querschnitt erstreckt, der sich weiter in einer ersten Dimension senkrecht zur Längsachse des Gehäuses erstreckt als in einer zur ersten Dimension senkrecht angeordneten zweiten Dimension, die auch als Dicke des Leitelements bezeichnet wird. Das Leitelement, das sich in der Düse beidseitig des Zuführungsrohrs erstreckt, begünstigt die Ausrichtung von Partikeln, die in dem Flüssigkeitsstrom, insbesondere in dem Kernstrom enthalten sind, insbesondere so, dass Partikel mit gestrecktem Querschnitt in eine gemeinsame Ausrichtung gebracht werden, bevorzugt in eine Ausrichtung, in der die längere Erstreckung des Querschnitts der Partikel etwa parallel zur ersten Dimension des Leitelements angeordnet ist. Das Zuführungsrohr kann innerhalb des Leitelements angeordnet sein oder als Bohrung innerhalb des Leitelements ausgebildet sein. Das Leitelement erstreckt sich daher in der ersten Dimension bis in einen geringeren Abstand zur Innenwand des Gehäuses, während es in der zweiten Dimension weiter von der Innenwand des Gehäuses beabstandet ist. Das Leitelement endet in einer Kante, die das Leitelement in seiner ersten Dimension begrenzt und die Dicke der zweiten Dimension aufweist. Das Leitelement bildet mit der Innenwand des Gehäuses einen lichten Querschnitt, der in zwei durch das Leitelement beabstandete Anteile geteilt ist, die sich bevorzugt ausschließlich in dem Bereich des lichten Querschnitts kontaktieren bzw. zusammenlaufen, um den das Leitelement in der ersten Dimension von der Innenwand des Gehäuses beabstandet ist. Das Leitelement weist eine Dicke auf, die sich in der zweiten Dimension seines Querschnitts erstreckt, die geringer als seine Erstreckung entlang seiner ersten Dimension ist. In der zweiten Dimension kann sich das Leitelement z.B. zu maximal 70%, bevorzugt maximal 50%, bevorzugter maximal 30%, maximal 20% oder maximal 10% im Verhältnis zur Erstreckung in der ersten Dimension erstrecken. In der zweiten Dimension kann sich der Querschnitt des Leitelements entlang der ersten Dimension verändern, insbesondere sich entlang der ersten Dimension von der Längsachse des Gehäuses bis zu seiner Kante verringern oder vergrößern.

Entlang der Längsachse des Gehäuses kann sich das Leitelement in seiner ersten Dimension bis in den gleichen Abstand von der Längsachse des Gehäuses bzw. bis zur Innenwand des Gehäuses erstrecken, oder sich mit längs der Längsachse des Gehäuses unterschiedlichem Abstand erstrecken, bzw. bis in einen unterschiedlichen Abstand von der Innenwand des Gehäuses. Der Querschnitt des Leitelements kann daher in seiner ersten Dimension in einer Kante enden, die längs der Längsachse des Gehäuses im gleichen Abstand zur Innenwand des Gehäuses angeordnet ist, oder längs der Längsachse des Gehäuses in unterschiedlichem Abstand von der Innenwand des Gehäuses. Z.B. kann die Kante in einem an das erste Ende des Leitelements angrenzenden Abschnitt in geringerem Abstand von der Innenwand des Gehäuses angeordnet sein als in einem daran angrenzenden Abschnitt, der der Austrittsöffnung des Zuführungsrohrs gegenüberliegt. Die Kante kann z.B. in dem an das erste Ende des Leitelements angrenzenden Abschnitt in einem längs der Längsachse des Gehäuses gleichen Abstand zur Innenwand des Gehäuses angeordnet sein und in dem angrenzenden Abschnitt, der der Austrittsöffnung gegenüberliegt, mit einem Abstand zur Innenwand des Gehäuses, der mit zunehmendem Abstand von der Austrittsöffnung größer wird. Optional ist die Kante abschnittsweise gradlinig oder bogenförmig, konvex oder konkav in Bezug zur Längsachse des Gehäuses. In Bezug zur zweiten Dimension kann die Kante plan, konkav oder konvex sein, insbesondere parallel zur Innenwand des Gehäuses.

Für die Erzeugung eines Flüssigkeitstroms mit tierischen Zellen als Partikel in einer Kernstromflüssigkeit kann sich die Innenwand des Gehäuses z.B. von einem Durchmesser von ca. 4 bis 10mm bis zum Auslass verjüngen, der z.B. einen Durchmesser von 0,2 bis 1 oder bis 0,5mm haben kann. Das Leitelement kann sich z.B. entlang der Längsachse des Gehäuses über 3 bis 5mm erstrecken und in der ersten Dimension von etwa 0,5mm bis 1,5 mm am ersten Ende bis etwa 3 bis 5mm maximal, während es sich in der zweiten Dimension etwa um 0,5 bis 1mm erstreckt, jeweils beidseitig von der Längsachse.

Das Leitelement ist bevorzugt zur Längsachse des Gehäuses symmetrisch und besteht insbesondere aus zwei Anteilen, die sich mit ihrer ersten Dimension in einer gemeinsamen Ebene erstrecken, in der die Längsachse des Gehäuses angeordnet ist, wobei weiter bevorzugt der Querschnitt des Leitelements entlang der Längsachse des Gehäuses symmetrisch ausgebildet ist.

Das Leitelement bewirkt eine Ausrichtung der in der Kernstromflüssigkeit enthaltenen Partikel nach deren Form in dem Abschnitt zwischen der Austrittsöffnung des Zuführungsrohrs und dem Auslass der Düse. Diese Ausrichtung der Partikel wird gegenwärtig darauf zurückgeführt, dass die Erstreckung des Leitelements in seiner zweiten Dimension zu unterschiedlichen Strömungsgeschwindigkeiten der Hüllstromflüssigkeit führt, insbesondere über die erste Dimension des Leitelements. Es wird angenommen, dass diese unterschiedlichen Strömungsgeschwindigkeiten zu einer Ausrichtung der Partikel führen, die diese im Wesentlichen auch nach Austritt aus dem Auslass der Düse aufweisen.

Die Düse hat den Vorteil, dass die Innenwand bzw. der Querschnitt ihres Gehäuses rotationssymmetrisch sein kann und daher auf einfache Weise herstellbar ist, z.B. mittels Bohren. Entsprechend verjüngt sich das Gehäuse dadurch, dass die Innenwand einen sich auf den Auslass verringernden Querschnitt aufspannt, der insbesondere rotationssymmetrisch um die Längsachse des Gehäuses ausgebildet ist. Bevorzugt ist der von der Innenwand des Gehäuses aufgespannte Querschnitt angrenzend an den Auslass kegelförmig. Optional kann der von der Innenwand des Gehäuses aufgespannte Querschnitt über den Abschnitt, der sich zwischen der Austrittsöffnung des Zuführungsrohrs und dem Auslass des Gehäuses erstreckt, sich verjüngen, insbesondere kegelförmig zulaufen. Optional kann der von der Innenwand des Gehäuses aufgespannte Querschnitt, der sich über den Abschnitt zwischen der Austrittsöffnung des Zuführungsrohrs und dem zweiten Ende des Leitelements oder bis an das zweite Ende des Gehäuses erstreckt, insbesondere angrenzend an den Abschnitt, der sich zwischen der Austrittsöffnung des Zuführungsrohrs und dem Auslass des Gehäuses erstreckt, sich verjüngen, insbesondere kegelförmig zulaufen, oder eine andere Form aufweisen, z.B. zylindrisch sein. Auch der Auslass kann rotationssymmetrisch sein, insbesondere eine runde Bohrung. Bevorzugt weist die Düse an ihrem ersten Ende einen Einsatz aus hartem Material auf, z.B. Keramik oder Saphir, in dem eine Bohrung als Auslass gebildet ist. Die Ausbildung des lichten Querschnitts der Düse, der die Strömung der Hüllstromflüssigkeit steuert, erfolgt durch das in dem Gehäuse angeordnete Leitelement, wobei der Querschnitt durch Formung der Außenfläche des Leitelements erzeugt wird, bzw. durch die unterschiedliche Erstreckung des Leitelements in seiner ersten und zweiten Dimension, die senkrecht zur Längsachse und senkrecht zueinander stehen. Die Düse hat daher den Vorteil, dass die nicht rotationssymmetrische Oberfläche des Leitelements als Außenfläche herstellbar ist, während die Innenwand des Gehäuses als rotationssymmetrische Fläche herstellbar ist.

Das Leitelement erstreckt sich von seinem ersten Ende bis zu seinem entlang der Längsachse des Gehäuses gegenüberliegenden zweiten Ende, wobei das erste Ende angrenzend an die Austrittsöffnung des Zuführungsrohrs oder in einem geringen Abstand weiter von dem Auslass des Gehäuses angeordnet ist, als die Austrittsöffnung des Zuführungsrohrs, z.B. um bis zu 10%, bevorzugt um bis zu 5% oder 2% des Abstands der Austrittsöffnung des Zuführungsrohrs vom Auslass des Gehäuses. Bevorzugt ist das erste Ende in der Ebene angeordnet, in der die Austrittsöffnung des Zuführungsrohrs liegt. Das zweite Ende des Leitelements kann an das zweite Ende des Gehäuses angrenzen oder kann in einem Abstand vom zweiten Ende des Gehäuses angeordnet sein, z.B. in einem Abstand von 1 bis 80%, bevorzugt 10 bis 50% der Erstreckung des Gehäuses von seinem Auslass zu seinem zweiten Ende oder zum zweiten Ende des Leitelements.

Bevorzugt erstreckt sich das Leitelement in seiner ersten und zweiten Dimension senkrecht zum Zuführungsrohr und entlang der Längsachse des Gehäuses, wobei insbesondere das Zuführungsrohr koaxial zur Längsachse des Gehäuses angeordnet ist.

Bevorzugt weist die Düse an der Eintrittsöffnung des Zuführungsrohrs einen Pufferbehälter für Kernstromflüssigkeit auf, in die eine Zuleitung für partikelhaltige Kernstromflüssigkeit mündet. Ein solcher Pufferbehälter erhöht den Anteil von Partikeln, die durch das Gehäuse in eine vorbestimmte Ausrichtung angeordnet werden. Dies wird gegenwärtig darauf zurückgeführt, dass ein Pufferbehälter Strömungseinflüsse aus der Zuleitung verringert, die sich ins Zuführungsrohr fortsetzen. Weiter bevorzugt weist die Düse einen Schwingungserzeuger auf, der an einer Wandung des Pufferbehälters für Kernstromflüssigkeit befestigt ist, die der Eintrittsöffnung des Zuführungsrohrs gegenüberliegt. Der Schwingungserzeuger ist bevorzugt ein mit elektrischer Spannung beaufschlagbares Piezoelement. Besonders bevorzugt ist der Schwingungserzeuger, z.B. das Piezoelement, unter Vorspannung gegen die Wand der Pufferkammer für Kernstromflüssigkeit befestigt, z.B. mit einem zwischen dem Schwingungserzeuger und dem Innenvolumen der Pufferkammer angeordneten Deckel gegen die Wand der Pufferkammer belastet.

Bevorzugt weist die Düse an der Einlassöffnung der Düse für Hüllstromflüssigkeit einen Pufferbehälter für Hüllstromflüssigkeit auf, der optional angrenzend an den Pufferbehälter für Kernstromflüssigkeit angeordnet ist, z.B. zwischen dem Pufferbehälter für Kernstromflüssigkeit und dem zweiten Ende des Gehäuses, wobei weiter optional das Zuführungsrohr durch den Pufferbehälter für Hüllstromflüssigkeit geführt ist. Ein Pufferbehälter für Hüllstromflüssigkeit verringert Einflüsse der Zuleitung von Hüllstromflüssigkeit auf die Strömung der Hüllstromflüssigkeit im lichten Querschnitt des Gehäuses und erhöht die Ausrichtung von Partikeln in eine vorbestimmte Orientierung.

Das Zuführungsrohr hat bevorzugt einen kreisförmigen Innenquerschnitt, der sich entlang der Längsachse des Gehäuses verjüngen kann und bevorzugt entlang der Längsachse des Gehäuses konstant ist.

Das Piezoelement dient als Schwingungserzeuger, der bevorzugt senkrecht zur Längsachse des Gehäuses verlaufende Druckwellen erzeugt, um bei Anordnung des Auslasses des Gehäuses in einem gasgefüllten Raum einen Tropfenstrom zu erzeugen.

Ein Durchflusszytometer mit der erfindungsgemäßen Düse weist bevorzugt zumindest eine erste Strahlungsquelle auf, die auf einen ersten Abschnitt des aus dem Gehäuse der Düse austretenden Flüssigkeitsstroms gerichtet ist, z.B. einen Laser, und einen gegenüber der Strahlungsquelle auf den ersten Abschnitt des Flüssigkeitsstroms gerichteten ersten Detektor, wobei optional der Detektor ein Signal erzeugt, das eine Auslenkungseinrichtung ansteuert, um Abschnitte des Flüssigkeitsstroms in Abhängigkeit von der Detektion mittels des Signals abzulenken, z.B. zu fraktionieren. Optional weist die Vorrichtung eine zweite Strahlungsquelle auf, die z.B. auf einen zweiten Abschnitt des Flüssigkeitsstroms zwischen der ersten Strahlungsquelle und der Düse gerichtet ist, und einen zweiten Detektor, der auf diesen zweiten Abschnitt gerichtet ist. Bevorzugt erzeugt der zweite Detektor ein zweites Signal, das die Auslenkungseinrichtung steuert, so dass Abschnitte des Flüssigkeitsstroms zusätzlich in Abhängigkeit von dem zweiten Signal ausgelenkt werden.

Die Auslenkungseinrichtung kann ein Paar elektrisch entgegengesetzt geladener Platten sein, die beidseitig des Flüssigkeitsstroms angeordnet sind, und einen elektrischen Kontakt aufweisen, der in der Düse angeordnet ist, insbesondere in dem Gehäuse. Optional kann der Kontakt das Zuführungsrohr für Kernstromflüssigkeit sein. Vorzugsweise ist der elektrische Kontakt in Abhängigkeit vom ersten und/oder zweiten Signal gesteuert, so dass eine positive oder negative Aufladung in Abhängigkeit vom ersten und/oder zweiten Signal erfolgt. Alternativ kann die Auslenkungseinrichtung ein auf den Flüssigkeitsstrom gerichteter Laser sein, der auf den Flüssigkeitsstrom gerichtet ist und eingerichtet ist, den Flüssigkeitsstrom nur oberflächlich bis zur oberflächlichen Verdampfung des Flüssigkeitsstroms zu verdampfen, wie dies z.B. in der WO2010/149739 beschrieben ist.

Das erfindungsgemäße Verfahren unter Verwendung der Düse bzw. eines Durchflusszytometers mit der Düse weist die folgenden Schritte auf:
- Bereitstellen einer partikelhaltigen Kernstromflüssigkeit,
- Bereitstellen einer Hüllstromflüssigkeit,
- Pumpen der Hüllstromflüssigkeit durch die Einlassöffnung für Hüllstromflüssigkeit,
- Pumpen der partikelhaltigen Kernstromflüssigkeit durch die Eintrittsöffnung des Zuführungsrohrs, wobei sich entlang der Längsachse des Gehäuses ein Leitelement erstreckt, wobei bevorzugt das Zuführungsrohr koaxial zur Längsachse des Gehäuses angeordnet ist, wobei das Leitelement den lichten Querschnitt des Innenvolumens des Gehäuses in zwei Teile unterteilt, und wobei das Leitelement sich entlang der Längsachse in größerem Maß entlang einer zur Längsachse senkrechten ersten Dimension bis in einen Abstand zur Innenwand des Gehäuses erstreckt als es sich in einer zur Längsachse und zur ersten Dimension senkrechten zweiten Dimension erstreckt,
- Durchströmenlassen des lichten Querschnitts des Gehäuses von Hüllstromflüssigkeit und Durchströmenlassen des Zuführungsrohrs von Kernstromflüssigkeit, wobei die Kernstromflüssigkeit nach Austritt aus der Austrittsöffnung des Zuführungsrohrs von der Hüllstromflüssigkeit kontaktiert wird und die in der Kernstromflüssigkeit enthaltenen Partikel in eine vorbestimmte Ausrichtung bewegt werden,
- optional Durchströmenlassen eines sich verjüngenden Abschnitts zwischen der Austrittsöffnung des Zuführungsrohrs und dem Auslass des Gehäuses, wodurch der Querschnitt von Hüllstromflüssigkeit und Kernstromflüssigkeit verringert wird, und
- Austretenlassen der von einer Hüllstromflüssigkeit umgebenen Kernstromflüssigkeit durch den Auslass des Gehäuses.
- Optional enthält das Verfahren den Schritt des Detektierens einer Eigenschaft der Partikel und,
- weiter optional, den Schritt des Behandelns (z.B. durch Laserbestrahlung) und/oder des Ablenkens der Partikel in getrennte Fraktionen bzw. Behälter in Abhängigkeit von einer detektierten Eigenschaft.
- Bevorzugt ist das Gehäuse rotationssymmetrisch.

Insbesondere bevorzugt ist ein Verfahren zur Herstellung geschlechtschromosomenspezifisch sortierter Fraktionen nicht-menschlicher Spermien, bei dem die Spermien durch die Düse in eine vorbestimmte Ausrichtung bewegt werden und in dieser Ausrichtung vor dem Strahlengang eines Detektors bewegt und detektiert werden.

Optional ist das Leitelement auf das Zuführungsrohr aufgeschoben, bevorzugt reversibel bzw. lösbar aufgeschoben, und z.B. am zweiten Ende des Gehäuses festgelegt. Bevorzugt ist das Leitelement einstückig mit dem Zuführungsrohr ausgebildet und an einem Ende gegenüber seiner Austrittsöffnung befestigt, z.B. mittels Eingriffs mit einem Deckel, der das Innenvolumen des Gehäuses an dessen zweitem Ende begrenzt.

Das Zuführungsrohr kann einen kreisförmigen Innenquerschnitt aufweisen. Alternativ kann das Zuführungsrohr einen gestreckten Innenquerschnitt aufweisen, z.B. einen elliptischen oder rechteckigen Innenquerschnitt, z.B. mit gerundeten Innenkanten, wobei die längere Erstreckung eines gestreckten Innenquerschnitts bevorzugt etwa parallel zur ersten Dimension angeordnet ist. Bevorzugt weist das Leitelement eine Symmetrieebene auf, durch die die Längsachse geht, und besonders bevorzugt erstreckt sich der Innenquerschnitt des Zuführungsrohrs in einer mit dem Leitelement gemeinsamen Symmetrieebene. Ein gestreckter Innenquerschnitt des Zuführungsrohrs kann z.B. ein Verhältnis der langen zur kurzen Erstreckung von maximal 0,3 oder maximal 0,2 aufweisen.

Die Düse, bevorzugt mit einem Einsatz aus Keramik oder Saphir, der den Auslass am ersten Ende des Gehäuses bildet, kann aus Kunststoff bestehen, z.B. aus PEEK oder POM, optional aus Keramik. Das Zuführungsrohr kann aus Metall bestehen und das Leitelement aus Kunststoff, z.B. aus PEEK oder POM; bevorzugt ist das Zuführungsrohr als Bohrung runden Querschnitts in dem Leitelement ausgebildet, das aus Kunststoff besteht, z.B. aus PEEK oder POM. Bevorzugt ist das Verfahren ein Verfahren zur Herstellung von Fraktionen nichtmenschlicher Säugetierspermien und weist den Schritt auf, nach dem Durchströmen des Zuführungsrohrs der Düse mit einer Kernstromflüssigkeit, die nichtmenschliche Spermien eines Individuums enthält, das Zuführungsrohr und das Leitelement gegen ein anders Zuführungsrohr und Leitelement zu tauschen oder zu sterilisieren, bevor Kernstromflüssigkeit, die nichtmenschliche Spermien eines anderen Individuums enthält, durch das Zuführungsrohr der Düse strömen gelassen wird.

### Genaue Beschreibung der Erfindung

Die Erfindung wird nun genauer anhand von Beispielen und mit Bezug auf die Figuren beschrieben, die schematisch in
- Figur 1 eine erfindungsgemäße Düse im Querschnitt längs der Längsachse der Düse,
- Figur 2 das in der Düse angeordnete Leitelement im Querschnitt senkrecht zur Längsachse der Düse,
- Figur 3 eine erfindungsgemäße Düse und
- Figur 4 A)-D) Leitelemente zeigen.

In den Figuren bezeichnen gleiche Bezugsziffern funktionsgleiche Elemente.

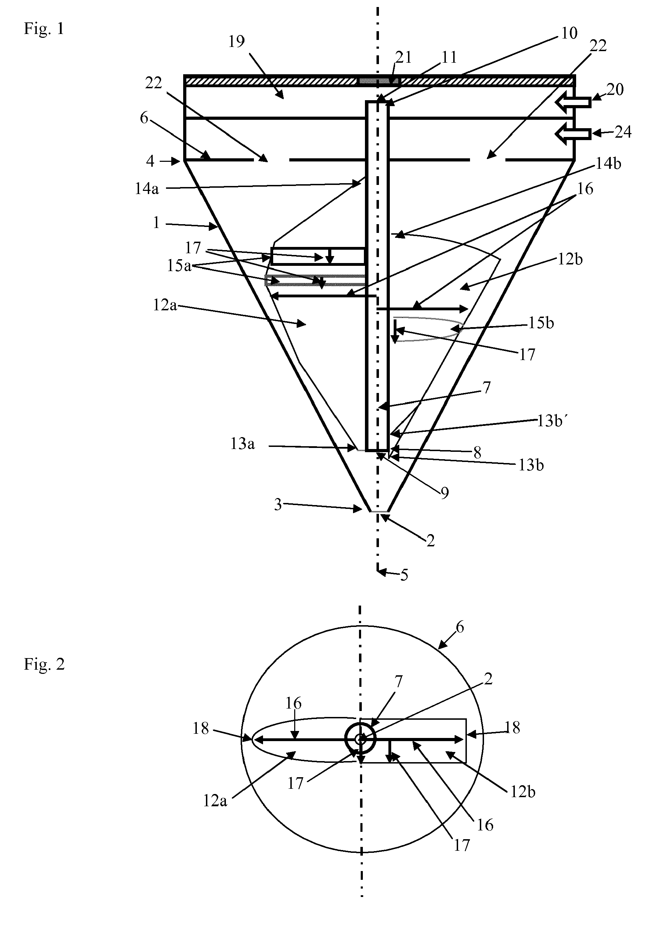

Figur 1 zeigt eine Düse mit einem Gehäuse 1, das sich von einem runden Auslass 2 an seinem ersten Ende 3 zu seinem gegenüberliegenden zweiten Ende 4 entlang einer Längsachse 5 erstreckt. Das zweite Ende 4 ist von einem Deckel 6 überdeckt, durch den sich das Zuführungsrohr 7 koaxial zur Längsachse 5 erstreckt. Das Zuführungsrohr 7 mündet an seinem ersten Ende 8 in einer Austrittsöffnung 9 und weist an seinem gegenüberliegenden zweiten Ende 10 eine Eintrittsöffnung 11 für eine partikelhaltige Kernstromflüssigkeit auf.

Figur 1 zeigt das Leitelement 12a, 12b in verschiedenen Gestaltungen, die jeweils hälftig beidseits des Zuführungsrohrs 7 dargestellt sind, so dass sich eine Gestaltung jeweils symmetrisch zur Längsachse 5 erstreckt. Das Leitelement 12a, 12b erstreckt sich von seinem ersten Ende 13a, 13b, das vom ersten Ende 8 des Zuführungsrohrs 7 beabstandet ist, zu seinem zweiten Ende 14a, 14b. Am Beispiel des Leitelements 12b ist entsprechend der bevorzugten Ausführungsform gezeigt, dass sich dessen erstes Ende 13b am ersten Ende 8 des Zuführungsrohrs 7 befinden kann und bündig mit diesem abschließen kann. Das zweite Ende 14b des alternativen Leitelements 12b zeigt, dass sich ein Leitelement 12a, 12b in unterschiedlichem Ausmaß entlang der Längsachse 5 des Gehäuses 1 erstrecken kann, z.B. in einem Abstand weiter vom Auslass des Gehäuses 1 angeordnet sein kann als die Austrittsöffnung 9 oder dass das Leitelement 12b das Zuführungsrohr 7 überragen kann, wie am ersten Ende 13b gezeigt, oder z.B. dass dessen erstes Ende 13b' um einen geringen Abstand weiter vom Auslass des Gehäuses angeordnet ist. Die schematisch eingezeichneten Querschnitte 15a, 15b des Leitelements 12a, 12b erstrecken sich senkrecht zur Längsachse 5 in einer ersten Dimension 16, zu der sich die Dicke des Leitelements 12a, 12b in der dazu senkrechten zweiten Dimension 17 erstreckt. Wie an Querschnitt 15a beispielhaft gezeigt ist, kann das Leitelement 12a eine konstante Dicke in der zweiten Dimension 17 aufweisen, oder wie beispielhaft am Querschnitt 15b gezeigt ist, kann das Leitelement 12b einen Querschnitt 15b aufweisen, der sich in der zweiten Dimension 17 entlang der ersten Dimension 16 verändert, z.B. mit zunehmendem Abstand zur Längsachse 5 verringert.

In Figur 1 ist gezeigt, dass sich das Leitelement 12a, 12b insbesondere in einem Abschnitt, der an sein erstes Ende 13a, 13b angrenzt, entlang der ersten Dimension 16 von der Längsachse 5 bis in einen etwa konstanten Abstand von der Innenfläche des Gehäuses 1 erstreckt, bzw. sich bis zu einer Kante 18 in einem etwa konstanten Abstand von der Innenfläche des Gehäuses 1 erstreckt.

Entsprechend der bevorzugten Ausführungsform zeigt Figur 1 eine Düse mit einem Pufferbehälter 19 für Kernstromflüssigkeit, in dem eine Zuführleitung 20 für Kernstromflüssigkeit mündet und an dem die Eintrittsöffnung 11 des Zuführungsrohrs 7 angeschlossen ist. Ein Piezoelement 21 ist unter Vorspannung in dem Pufferbehälter 19 für Kernstromflüssigkeit gegenüber der Eintrittsöffnung 11 des Zuführungsrohrs 7 befestigt. Weiter ist ein Pufferbehälter 22 für Hüllstromflüssigkeit mit dem Einlass 23 für Hüllstromflüssigkeit verbunden, die in das Gehäuse 1 münden, wobei der Pufferbehälter 22 für Hüllstromflüssigkeit mit einer Zuführleitung 24 für Hüllstromflüssigkeit verbunden ist. Der Pufferbehälter 22 für Hüllstromflüssigkeit ist zwischen dem zweiten Ende 4 der Düse 1 und dem Pufferbehälter 19 für Kernstromflüssigkeit angeordnet, wobei das Zuführungsrohr 7 durch den Pufferbehälter 22 für Hüllstromflüssigkeit angeordnet ist.

Figur 2 zeigt senkrecht zur Längsachse 5 der Düse bzw. des Gehäuses 1 liegende Querschnitte 15a, 15b durch das Leitelement 12a, 12b, wobei nur eine Hälfte des Leitelements 12a, 12b gezeigt ist, das sich spiegelsymmetrisch zur zweiten Dimension 17 bzw. zur Längsachse 5 erstreckt. Bevorzugt ist das Zuführungsrohr 7 kreisförmig um die Längsachse 5 des Gehäuses 1 angeordnet, die im Schnittpunkt der ersten Dimension 16 und der zweiten Dimension 17 liegt. Wie generell bevorzugt, ist der Auslass 2 des Gehäuses 1 symmetrisch um die Längsachse 5 des Gehäuses 1 angeordnet.

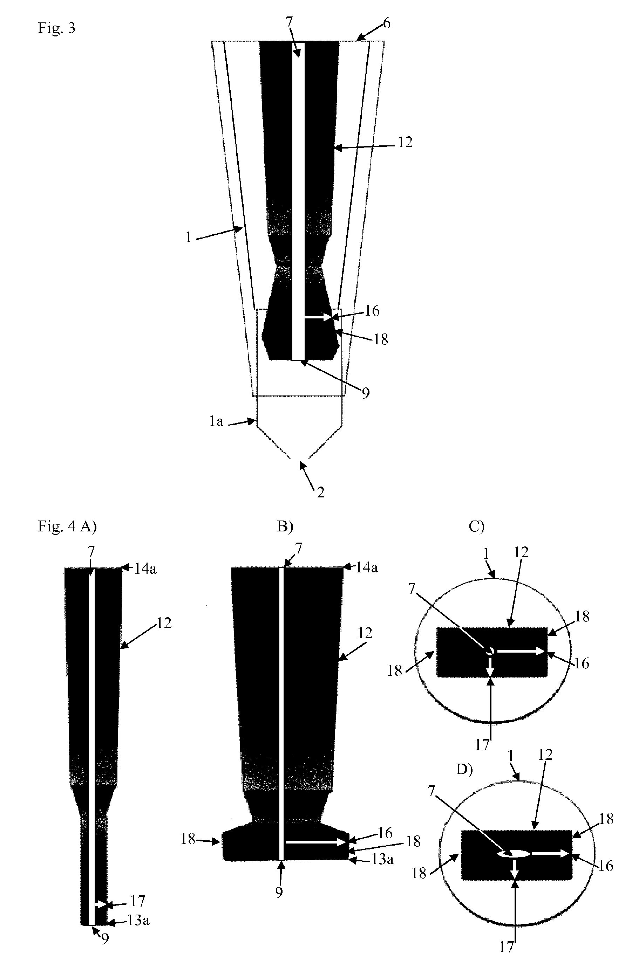

Figur 3 zeigt eine Düse, in deren Gehäuse 1 ein Leitelement 12a angeordnet ist, in dem das Zuführungsrohr 7 als Bohrung ausgeführt ist, die koaxial zur Längsachse 5 des Gehäuses 1 angeordnet ist. Der Auslass 2 des Gehäuses 1 ist durch einen Einsatz 1a gebildet, der in einem an den Auslass 2 angrenzenden Abschnitt kegelstumpfförmig ist und daran angrenzend zylindrisch.

Figur 4A) zeigt das Leitelement 12a von Figur 3 in verkleinerter Darstellung um 90° um die Längsachse 5 gedreht. Aus Figur 3 und 4A) wird deutlich, dass sich das Leitelement 12a in der zur Längsachse 5 senkrechten ersten Dimension 16 weiter erstreckt, als in der zur Längsachse 5 und zur ersten Dimension 16 senkrechten zweiten Dimension 17.

Figur 4B) zeigt eine optionale Ausführung eines Leitelements 12a, das sich in einem an die Austrittsöffnung 9 angrenzenden Abschnitt wesentlich weiter in seiner ersten Dimension 16 erstreckt als in seiner zweiten Dimension 17, die senkrecht zur Bildebene liegt, während der Querschnitt des Leitelements 12a in einem angrenzenden Abschnitt, der von der Austrittsöffnung 9 beabstandet ist, in der ersten Dimension 16 verjüngt ist.

Figur 4C) zeigt einen zur Längsachse 5 des Gehäuses senkrechten Querschnitt 15a eines Leitelements 12a, in dem das Zuführungsrohr 7 als zentrale Bohrung mit rundem Querschnitt ausgebildet ist.

Figur 4D) zeigt ein Leitelement entsprechend Figur 4C), bei dem das Zuführungsrohr 7 einen elliptischen Innenquerschnitt aufweist, dessen lange Erstreckung parallel zu der ersten Dimension 16 liegt und dessen kurze Erstreckung parallel zur zweiten Dimension 17 liegt. Überdies zeigt Figure 4D), dass der Innenquerschnitt des Zuführungsrohrs 7 dieselbe Symmetrieebene aufweist, die durch die Längsachse (senkrecht zur Darstellungsebene) geht, wie der Querschnitt des Leitelements 12a.

### Beispiel 1: Detektion Y-Chromosomen-haltiger Spermien in Frischsamen und geschlechtsspezifische Sortierung

Frisch gewonnener Bullensamen wurde in üblicher Weise in Verdünner verdünnt und mit DNA-spezifischem Farbstoff, z.B. Bisbenzimid H 33342 (Hoechst), für 30 bis 60 min bei einer Temperatur von 20 °C bis 40 °C inkubiert und anschließend in einem Durchflusszytometer gemäß US 5125759 oder der DE 10 2005 044 530 mit Licht der Anregungswellenlänge für den Farbstoff bestrahlt. Die jeweilige Emission wurde gemessen.

Die Ausrichtung der Spermien wurde mit einem Detektor bestimmt, der unmittelbar stromabwärts der Düse auf den austretenden Flüssigkeitsstrom aus vereinzelten Tropfen gerichtet war. Der Gesamt-DNA-Gehalt wurde mit einem weiteren Detektor bestimmt, der weiter stromabwärts auf den Flüssigkeitsstrom gerichtet war. Die Ablenkungseinrichtung wies zwei entgegengesetzt geladene Platten beidseitig des Flüssigkeitsstroms auf und einen Kontakt zur elektrischen Aufladung der Flüssigkeit in der Düse. Diese Aufladung wurde wie bekannt in Abhängigkeit von dem Signal des Detektors aufgegeben, der die Ausrichtung der Spermien bestimmt, und die Polarität der Aufladung in Abhängigkeit von dem Signal des Detektors zur Bestimmung des Gesamt-DNA-Gehalts. Auf diese Weise wurden die Spermatozoen abhängig von dem detektierten Signal durch ein elektrisches Feld in geschlechtschromosomspezifische Fraktionen abgelenkt.

Optional wurde ein Fluorid zur Immobilisierung der Spermien zugesetzt, z.B. in die während des Sortierverfahrens verwendete Hüll- oder Transportflüssigkeit, und/oder vor oder während des Zusatzes des Farbstoffs, um die Penetration des Farbstoffs in die Spermatozoen zu erhöhen. Fluoridionen wurden im Bereich von 0,1 bis 100 mM, vorzugsweise von 10 nM bis 10 mM zugesetzt. Es wurde gefunden, dass die optimale Konzentration des Fluorids, z.B. NaF oder KF, zwischen verschiedenen Spezies und für Individuen abwich. Die optimale Konzentrationen für die Spezies ist spezifisch und konnte allgemein als die Konzentration bestimmt werden, die bei mikroskopischer Betrachtung eine Immobilisierung von wenigstens 90% der Spermatozoen ergab, vorzugsweise von im wesentlichen allen Spermatozoen. Entsprechend bezieht sich die vorliegende Erfindung auch auf Zusammensetzungen der mit dem erfindungsgemäßen Verfahren hergestellten Spermafraktionen, und auf Verfahren zur Herstellung von geschlechtsspezifischen Spermafraktionen und anschließenden Konservierung der Spermafraktionen von nicht-menschlichen Säugetieren, jeweils vorzugsweise in Anwesenheit von Fluorid und/oder Antioxidationsmitteln.

**Bezugszeichenliste:**

| | |
|---|---|
| 1 | Gehäuse |
| 1a | Einsatz |
| 2 | Auslass |
| 3 | erstes Ende des Gehäuses |
| 4 | zweites Ende des Gehäuses |
| 5 | Längsachse |
| 6 | Deckel |
| 7 | Zuführungsrohr |
| 8 | erstes Ende des Zuführungsrohrs |
| 9 | Austrittsöffnung |
| 10 | zweites Ende des Zuführungsrohrs |
| 11 | Eintrittsöffnung |
| 12a, 12b | Leitelement |
| 13a, 13b, 13b' | erstes Ende des Leitelements |
| 14a, 14b | zweites Ende des Leitelements |
| 15a, 15b | Querschnitt des Leitelements |
| 16 | erste Dimension |
| 17 | zweite Dimension |
| 18 | Kante |
| 19 | Pufferbehälter für Kernstromflüssigkeit |
| 20 | Zuführleitung für Kernstromflüssigkeit |
| 21 | Piezoelement |
| 22 | Pufferbehälter für Hüllstromflüssigkeit |
| 23 | Einlass für Hüllstromflüssigkeit |
| 24 | Zuführleitung für Hüllstromflüssigkeit |

## Patentansprüche

1. Düse für ein Durchflusszytometer mit einem Gehäuse (1), dessen Innenquerschnitt sich entlang seiner Längsachse (5) von seinem zweiten Ende (4) zu einem Auslass (2) an seinem ersten Ende (3) verjüngt und in dem ein Zuführungsrohr (7) für eine Kernstromflüssigkeit angeordnet ist, das eine Eintrittsöffnung (11) aufweist und das in einer an seinem ersten Ende (8) angeordneten Austrittsöffnung (9) in einem Abstand zum Auslass (2) mündet, wobei ein Einlass (23) für eine Hüllstromflüssigkeit an dem Gehäuse (1) angeordnet ist,
**gekennzeichnet durch**
ein Leitelement (12), das sich entlang der Längsachse (5) in größerem Maß entlang einer zur Längsachse (5) senkrechten ersten Dimension (16) bis in einen Abstand zur Innenwand des Gehäuses (1) erstreckt als es sich in einer zur Längsachse (5) und zur ersten Dimension (16) senkrechten zweiten Dimension (17) erstreckt.

2. Düse nach Anspruch 1, **dadurch gekennzeichnet, dass** sich das Leitelement (12) von seinem ersten Ende (13), das in der Ebene des ersten Endes (8) des Zuführungsrohrs (7) liegt oder um einen Abstand gegenüber vom ersten Ende (8) des Zuführungsrohrs versetzt ist, bis zu seinem zweiten Ende (14) erstreckt, das in der Ebene des zweiten Endes (4) der Düse (1) liegt oder in einem Abstand vom zweiten Ende (4) der Düse (1) angeordnet ist.

3. Düse nach einem der voranstehenden Ansprüche, **dadurch gekennzeichnet, dass** das erste Ende (13a) des Leitelements (12a) in der Ebene angeordnet ist, in der sich die Austrittsöffnung (9) erstreckt.

4. Düse nach einem der voranstehenden Ansprüche, **dadurch gekennzeichnet, dass** sich die zweite Dimension (17) des Querschnitts (15a, 15b) des Leitelements (12, 12a, 12b) mit zunehmendem Abstand von der Längsachse (5) verringert.

5. Düse nach einem der voranstehenden Ansprüche, **dadurch gekennzeichnet, dass** das Leitelement (12) sich in einem an sein erstes Ende (13) angrenzenden Abschnitt in der ersten Dimension (16) bis zu einer Kante (18) erstreckt, die parallel zur Innenwand des Gehäuses (1) verläuft.

6. Düse nach einem der voranstehenden Ansprüche, **dadurch gekennzeichnet, dass** das Gehäuse (1) eine zu seiner Längsachse (5) rotationssymmetrische Innenwand aufweist.

7. Düse nach einem der voranstehenden Ansprüche, **dadurch gekennzeichnet, dass** sich das Zuführungsrohr (7) koaxial zur Längsachse (5) erstreckt.

8. Düse nach einem der voranstehenden Ansprüche, **dadurch gekennzeichnet, dass** das Zuführungsrohr (7) einen gestreckten Innenquerschnitt aufweist, insbesondere einen ovalen oder rechteckigen Innenquerschnitt.

9. Düse nach einem der voranstehenden Ansprüche, **dadurch gekennzeichnet, dass** das Zuführungsrohr (7) als Bohrung in dem Leitelement (12, 12a, 12b) ausgebildet ist.

10. Düse nach einem der Ansprüche 1 bis 8, **dadurch gekennzeichnet, dass** das Zuführungsrohr (7) in dem Leitelement (12, 12a, 12b) befestigt ist.

11. Düse nach einem der voranstehenden Ansprüche, **dadurch gekennzeichnet, dass** das Leitelement (12, 12a, 12b) mit dem Zuführungsrohr (7) an einem Deckel (6) reversibel festlegbar ist, der das Gehäuse (1) an seinem zweiten Ende (4) überdeckt.

12. Düse nach einem der voranstehenden Ansprüche, **dadurch gekennzeichnet, dass** das sie einen Pufferbehälter (19) für Kernstromflüssigkeit aufweist, in den die Eintrittsöffnung (11) des Zuführungsrohrs (7) mündet.

13. Düse nach einem der voranstehenden Ansprüche, **dadurch gekennzeichnet, dass** das sie einen Pufferbehälter (22) für Hüllstromflüssigkeit aufweist, der mit dem Einlass (23) für Hüllstromflüssigkeit verbunden ist.

14. Verfahren zur Erzeugung eines Flüssigkeitsstroms mit einem von einem Hüllstrom umgebenen partikelhaltigen Kernstrom durch Eintretenlassen einer partikelhaltigen Kernstromflüssigkeit in eine Eintrittsöffnung (11) für Kernstromflüssigkeit und Eintretenlassen einer Hüllstromflüssigkeit in eine Einlassöffnung für Hüllstromflüssigkeit einer Düse (1) nach einem der voranstehenden Ansprüche.

15. Verfahren nach Anspruch 14, **dadurch gekennzeichnet, dass** die Partikel einen Querschnitt (15a, 15b) aufweisen, der in einer zur Längsachse (5) des Gehäuses (1) der Düse senkrechten ersten Dimension (16) kleiner ist als in einer zur Längsachse (5) und zur ersten Dimension (16) senkrechten zweiten Dimension (17).

16. Verfahren nach einem der Ansprüche 14 bis 15, **dadurch gekennzeichnet, dass** in dem aus der Düse austretenden Flüssigkeitsstrom zumindest eine Eigenschaft der in dem Kernstrom enthaltenen Partikel detektiert wird und die Partikel abhängig von der detektierten Eigenschaft in zumindest zwei Fraktionen getrennt werden, oder in Abhängigkeit von der detektierten Eigenschaft unterschiedlich behandelt werden.

17. Verfahren nach einem der Ansprüche 14 bis 16 zur Herstellung eines Präparats von Partikeln, die einen Querschnitt mit einer ersten Dimension und einer zweiten Dimension aufweisen, wobei die Partikel in der ersten Dimension kleiner sind als in der zweiten Dimension, **gekennzeichnet dadurch, dass** die Partikel nicht-menschliche Säugetierspermien sind, die detektierte Eigenschaft das Vorliegen des X-Chromosoms oder des Y-Chromosoms in dem Spermium ist, und durch das Bestrahlen der Spermien in Abhängigkeit von der detektierten Eigenschaft oder das Ablenken der Spermien in Abhängigkeit von der detektierten Eigenschaft in zumindest zwei unterschiedliche Fraktionen.
